# EUROPEAN PATENT SPECIFICATION

(11) **EP 3 458 049 B1**
(45) Date of publication and mention of the grant of the patent: **05.03.2025**
(21) Application number: 17727823.1
(22) Date of filing: 19.05.2017
(51) Int. Cl.: A61K 31/4015, A61P 25/00

(54) **LEVETIRACETAM, BRIVARACETAM OR SELECTRACETAM FOR USE IN TREATING SEPSIS INDUCED ACUTE BRAIN DYSFUNCTION**
LEVETIRACETAM, BRIVARACETAM ODER SELECTRACETAM ZUR VERWENDUNG BEI DER BEHANDLUNG VON SEPSIS-INDUZIERTER AKUTER HIRNDYSFUNKTION
LÉVÉTIRACÉTAM, BRIVARACÉTAM OU SÉLECTRACÉTAM POUR UNE UTILISATION DANS LE TRAITEMENT D'UN DYSFONCTIONNEMENT CÉRÉBRAL AIGU INDUIT PAR UNE SEPSIE

(30) Priority: 20.05.2016 US 201662339414 P
(43) Date of publication of application: 27.03.2019
(73) Proprietor: Institut Pasteur, 75724 Paris Cedex 15 (FR)
(72) Inventor: SHARSHAR, Tarek, 75724 Paris Cedex 15 (FR); CHRETIEN, Fabrice, 75008 Paris (FR); MAZERAUD, Aurélien, 75724 Paris Cedex 15 (FR); BOZZA, Fernando Augusto, 22451-041 Rio De Janeiro (BR)
(74) Representative: Plasseraud IP
(86) International application number: PCT/EP2017/062190
(87) International publication number: WO 2017/198862

(56) References cited:
- OYTUN ERBAS ET AL: "The Beneficial Effects of Levetiracetam on Polyneuropathy in the Early Stage of Sepsis in Rats: Electrophysiological and Biochemical Evidence", JOURNAL OF INVESTIGATIVE SURGERY., vol. 26, no. 6, 19 August 2013 (2013-08-19), GB, pages 312 - 318, XP055395171, ISSN: 0894-1939, DOI: 10.3109/08941939.2013.797056
- C. VOGL ET AL: "The Synaptic Vesicle Glycoprotein 2A Ligand Levetiracetam Inhibits Presynaptic Ca2+ Channels through an Intracellular Pathway", MOLECULAR PHARMACOLOGY, vol. 82, no. 2, 3 May 2012 (2012-05-03), pages 199 - 208, XP055395228, DOI: 10.1124/mol.111.076687
- SAMANTHA STERKEL ET AL: "Preserving brain function in a comatose patient with septic hyperpyrexia (41.6 °C): a case report", JOURNAL OF MEDICAL CASE REPORTS, vol. 11, no. 1, 1 January 2017 (2017-01-01), XP055395164, DOI: 10.1186/s13256-017-1204-8

## Description

### FIELD OF THE INVENTION

The present invention pertains to the field of sepsis prevention and/or treatment and relates to the use of a synaptic vesicle 2a binding chemical entity for preventing and/or treating sepsis induced acute brain dysfunction (SiBD) and/or reducing sepsis mortality as defined in the claims.

### BACKGROUND

Sepsis is an associated infection and systemic inflammation that ranges from isolated fever to multiple organ dysfunctions and/or septic shock. Sepsis affects 950,000 patients per year in Europe and the mortality varies from 26% to 60%, according to its severity. In 60% of cases, sepsis induces an acute brain dysfunction (SiBD) that is not related to direct brain infection and is clinically characterized by altered consciousness - ranging from confusion to coma and by various electroencephalographic (EEG) changes (Adam et al., Expert. Rev. Anti Infect. Ther., 2013, 11, 211-221). SiBD is complicated by increased mortality but also long-term psychological and cognitive dysfunctions. Attention, memory and executive functions are mainly impaired, linking SiBD to dementias; anxiety, depression and post-traumatic stress syndrome are frequent, linking SiBD also to psychiatric disorders.

SiBD results mainly from a excitotoxic and a neuro-inflammatory process of which networks involved in response to stress and activation of the microglial cells are two concurrent key components.

Pathologic examination of the brain of fatal cases of sepsis consistently exhibits increased apoptosis in specific structures (ie. the amygdala, nucleus tractus solitarii, and locus coeruleus). The kinetic of central nuclei activation has been described in a murine model of sepsis in which c-Fos expression (a transcription factor specific to neurons presenting an increased activity) was observed early in the above mentionned areas. These structures are known to activate in response to stress and are especially sensitive to hypoxia, leading to excitotoxic processes, structural changes, and neurologic dysfunction. Theses structural dysfunctions may be responsible for the cognitive impairment and psychiatric disorders of sepsis-associated brain dysfunction. To date no neuromodulatory strategy has proven efficient in human to prevent the neurological alterations observed after sepsis associated encephalopathy. Also sudden cardiac arrest is observed during sepsis without an identified cause.

Levetiracetam has been administered to septic rats (Erbas, Oytun, et al., "The Beneficial Effects of Levetiracetam on Polyneuropathy in the Early Stage of Sepsis in Rats: Electrophysiological and Biochemical Evidence," Journal of Investigative Surgery, Vol. 26, pp. 312-318 (2013)). Erbas induced sepsis in rats using a cecal ligation and puncture method. Levetiracetam was then administered one hour after surgery in a single dose of 300 mg/kg, 600 mg/kg, or 1200 mg/kg. Erbas analyzed the protective effects of levetiracetam in the peripheral nervous system and concluded that levetiracetam displays a broad spectrum of protective and restorative effects on the peripheral nervous system.

For these and other reasons there is a long-felt and unmet need to provide a chemical entity for use in methods of treatment for SiBD that will reduce or ameliorate at least one symptom or manifestation of SiBD. The present invention meets these and other needs.

### SUMMARY OF THE INVENTION

As demonstrated in the examples, the inventors have made the surprising discovery that administration of levetiracetam, an example of a synaptic vesicle 2a binding chemical entity, to a subject with sepsis can reduce the cognitive and physiological manifestations of SiBD in a subject with sepsis. The inventors have also discovered that administration of a synaptic vesicle 2a binding chemical entity to a subject with sepsis can reduce sepsis mortality, among other things.

Accordingly, the invention as set out in the appended set of claims is directed to (i) a synaptic vesicle 2a binding chemical entity for use in the prevention and/or treatment of sepsis induced acute brain dysfunction (SiBD), wherein the synaptic vesicle 2a binding chemical entity is levetiracetam, brivaracetam or selectracetam; and (ii) a synaptic vesicle 2a binding chemical entity for use for reducing sepsis mortality, wherein the synaptic vesicle 2a binding chemical entity is levetiracetam, brivaracetam or selectracetam, and wherein the synaptic vesicle 2a binding chemical entity is administered for at least one week.

In some embodiments of the uses of the synaptic vesicle 2a binding chemical entity as set out in the appended set of claims, the synaptic vesicle 2a binding chemical entity is levetiracetam.

In some embodiments of the uses of the the synaptic vesicle 2a binding chemical entity as set out in the appended set of claims, the synaptic vesicle 2a binding chemical entity is administered every twelve hours.

In some embodiments of the uses of the the synaptic vesicle 2a binding chemical entity as set out in the appended set of claims, the synaptic vesicle 2a binding chemical entity is administered for at least two weeks.

In some embodiments of the uses of the the synaptic vesicle 2a binding chemical entity as set out in the appended set of claims, the synaptic vesicle 2a binding chemical entity is administered for at least one month.

In some embodiments of the uses of the the synaptic vesicle 2a binding chemical entity as set out in the appended set of claims, administration of the synaptic vesicle 2a binding chemical entity to the subject increases the cerebral level of IL1β in the subject.

In some embodiments of the uses of the the synaptic vesicle 2a binding chemical entity invention as set out in the appended set of claims, administration of the synaptic vesicle 2a binding chemical entity to the subject increases the cerebral level of IL4 in the subject.

In some embodiments of the uses of the the synaptic vesicle 2a binding chemical entity invention as set out in the appended set of claims, administration of the synaptic vesicle 2a binding chemical entity to the subject increases hippocampal expression of lba-1 in the subject.

In some embodiments of the uses of the the synaptic vesicle 2a binding chemical entity invention as set out in the appended set of claims, administration of the synaptic vesicle 2a binding chemical entity to the subject reduces cleavage of Caspase 3 in the brain of the subject.

In some embodiments of the use of the synaptic vesicle 2a binding chemical entity in the prevention and/or treatment of sepsis induced acute brain dysfunction (SiBD) as set out in the appended set of claims, the synaptic vesicle 2a binding chemical entity is administered for at least two or four days.

In some embodiments of the use of the synaptic vesicle 2a binding chemical entity in the prevention and/or treatment of sepsis induced acute brain dysfunction (SiBD) as set out in the appended set of claims, the synaptic vesicle 2a binding chemical entity is administered for at least one week.

In some embodiments of the use of the synaptic vesicle 2a binding chemical entity in the prevention and/or treatment of sepsis induced acute brain dysfunction (SiBD) as set out in the appended set of claims, administration of the synaptic vesicle 2a binding chemical entity to the subject reduces development of at least one cognitive indicator of SiBD in the subject.

In some embodiments of the use the the synaptic vesicle 2a binding chemical entity in the prevention and/or treatment of sepsis induced acute brain dysfunction (SiBD) as set out in the appended set of claims, administration of the synaptic vesicle 2a binding chemical entity to the subject reduces development of post traumatic stress disorder induced by SiBD in the subject.

In some embodiments of the use the the synaptic vesicle 2a binding chemical entity in the prevention and/or treatment of sepsis induced acute brain dysfunction (SiBD) uses as set out in the appended set of claims, administration of the synaptic vesicle 2a binding chemical entity to the subject reduces the risk of sepsis induced death in the subject.

In some embodiments of the use the the synaptic vesicle 2a binding chemical entity in the prevention and/or treatment of sepsis induced acute brain dysfunction (SiBD) invention as set out in the appended set of claims, administration of the synaptic vesicle 2a binding chemical entity to the subject reduces the risk of sepsis induced dementia in the subject.

### BRIEF DESCRIPTION OF THE DRAWINGS

**Figure 1** shows that levetiracetam reduces death and cognitive and behavioural impairments after sepsis.
**Figure 2** shows innate anxiety and depression after sepsis and the effect of Levetiracetam.
**Figure 3** shows modulation of inflammatory markers by levetiracetam.
**Figure 4** shows an analysis of effects of levetiracetam on astrocytes and cell death.
**Figure 5** shows effect of levetiracetam on cytokine transcription in activated primary murine culture of microglial cells. Lipopolysaccharides at 1 and 10 ng/mL concentration, LPS1 and LPS10 respectively; Levetiracetam 10 and 100 microgram/mL, K10 and k100 respectively. A- IL6. **B**-TNF-alpha. **C-**IL1-beta
**Figure 6** shows pro- and anti-inflammatory markers transcription in primary murine microglial cells exposed to LPS and levetiracetam. **A**-ptsg2. **B**-Lgals3. **C**-Arg1. **D**-IL1Rn. **E-**IL4Ra.**F**-CD32. **G.** CD206.
**Figure 7** shows effect of levetiracetam on Inducible NO synthase gene (*inos*) transcription in activated primary murine microglial cells.
**Figure 8** shows effect of levetiracetam on Nitric Oxide production in murine primary monocytic cells exposed to LPS.

### DETAILED DESCRIPTION OF THE INVENTION

The invention is as defined in the claims.

### A. Introduction

Amygdala is an overactivated area that presents neuropathological lesions related to excitotoxicity during sepsis. Limbic epilepsia are observed in human and lead to sudden cardiac arrest.

The resident immune cells of the CNS, microglial cells, are normally quiescent but they can be activated by various factors. They are then able to proliferate, to migrate, to release various factors and to phagocyte. In addition to their role in immune defense and scavenging, microglial cells are also involved in the maintenance of synaptic network and blood brain barrier (BBB) integrity. Activation is characterized by changes in morphology and in immune status. The morphological phenotypes range from hyperamified to amoeboid forms, the latter reflecting the ultimate state of activation. The immunological phenotypes are commonly classified as pro or anti-inflammatory, which are respectively considered to be neurotoxic and neuroprotective.

Microglial activated "morphotype" is a consistent phenomenon in neuropathological studies of septic patients or animals. However, there is not a straightforward correlation between the morphological and immunological phenotypes. Moreover, morphological changes and immune polarization are dynamic processes that are regulated by various factors. The changes along the course of sepsis in microglial morphology and immune status have not been comprehensively assessed as well as their relationships with mitochondrial dysfunction. Indeed mitochondrial dysfunction can compromise the physiological activities and viability of the microglial cells. However, if mitochondrial dysfunction and oxidative stress have been experimentally evidenced on whole brain of septic mice, they have not been specifically documented in microglial cells. Finally, because of their potential neuroprotective and neurotoxic effects, pharmacological modulation of microglial cells is a major experimental and clinical issue that has been scantly addressed in sepsis.

Levetiracetam is an antiepileptic drug that is widely used in intensive care for epilepsia treatment and prevention. Its pharmacodynamy has been well described. Levetiracetam presents an excellent biodisponibility and crosses easily the blood brain barrier to reach the CNS. Levetiracetam binds to the synaptic vesicle 2a located on neurotransmitters vesicle's surface at the presynaptic terminal button. Once bond, neurotransmitter release is partially inhibited and the neurotransmitter's vesicle presynaptic pool renewal rate is decreased. Neurophysiological experiments did not show alterations of the resting state potential nor modifications in the spontaneous firing rate of neurons, but decreased in murine model the probability of epileptic activity generalization. This correlates with the clinical use of levetiracetam that is non-sedative, does not alter cognitive function or children development but is an efficient drug to treat various types of epilepsia. Besides epilepsia, levetiracetam has been successfully used in neuropathic pain. Neuropathic pain results in an abnormal increased activity in central nervous system neurons involved in pain.

Levetiracetam has been administered to septic rats (Erbas, Oytun, et al., cited previously).

Levetiracetam has also been administered to rats treated to model traumatic brain injury (Browning, Megan, et al., " Levetiracetam Treatment in Traumatic Brain Injury: Operation Brain Trauma Therapy," Journal of Neurotrauma, Vol. 33, pp. 581-594 (2016). Levetiracetam was administered at 54 mg/kg or 170 mg/kg in a single perfusion over a 15 minute period beginning 15 minutes after injury. Browning concluded that levetiracetam improved cognitive outcome in treatment groups.

Levetiracetam has not been administered to septic subjects over longer periods of time and has not been associated with improved cognitive outcomes and improved CNS outcomes in subjects following extended dosing. As demonstrated in the examples, the inventors have made the surprising discovery that administration of levetiracetam, a synaptic vesicle 2a binding chemical entity, to a subject with sepsis can reduce the cognitive and physiological manifestations of SiBD in a subject with sepsis.

### B. Synaptic Vesicle 2a Binding Proteins

Synaptic vesicle 2a (SV2A) belongs to the major facilitator superfamily (MFS) of transporters and are integral constituents of synaptic vesicle membranes. They have been demonstrated to be involved in vesicle trafficking and exocytosis, processes crucial for neurotransmission.

The synaptic vesicle 2a binding chemical entity is levetiracetam, brivaracetam (BRIV) or selectracetam (SEL).

Levetiracetam, a single enantiomer, is (-)-(S)-α-ethyl-2-oxo-1-pyrrolidine acetamide, its molecular formula is C₈H₁₄N₂O₂ and its molecular weight is 170.21. Levetiracetam has the following structural formula:

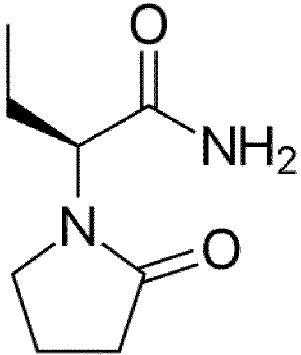

Levetiracetam is a white to off-white crystalline powder with a faint odor and a bitter taste. It is very soluble in water (104.0 g/100 mL). It is freely soluble in chloroform (65.3 g/100 mL) and in methanol (53.6 g/100 mL), soluble in ethanol (16.5 g/100 mL), sparingly soluble in acetonitrile (5.7 g/100 mL) and practically insoluble in n-hexane. (Solubility limits are expressed as g/100 mL solvent.)

In particular, the synaptic vesicle 2a binding chemical entity chosen from levetiracetam, brivaracetam (BRIV) or selectracetam (SEL), is administered orally in the form of solid or liquid compositions, for example in the form of tablets, pills, dragees, gelatine capsules, solutions or syrups. In particular, the synaptic vesicle 2a binding chemical entity chosen from levetiracetam, brivaracetam (BRIV) or selectracetam (SEL), is administered parenterally, e.g., intravenously, in the form of injectable solutions or suspensions.

Pharmaceutical forms such as solutions or tablets are prepared according to conventional pharmaceutical methods. The synaptic vesicle 2a binding chemical entity chosen from levetiracetam, brivaracetam (BRIV) or selectracetam (SEL), may be mixed with a solid or liquid non-toxic pharmaceutically acceptable carrier and optionally with a dispersant and/or a stabilizer. In some embodiments a colorant and/or sweetener is also included.

Suitable solid and liquid pharmaceutical carriers are well known in the art. Suitable solid pharmaceutical excipients for the preparation of tablets or capsules include, for example, starch, talc, calcium carbonate, lactose, sucrose and magnesium stearate.

Certain dosage forms of levetiracetam are marketed under various trade names, including KEPPRA^{®} among others. KEPPRA^{®} tablets contain 250 mg, 500 mg, 750 mg, or 1,000 mg of levetiracetam. Inactive ingredients include colloidal silicon dioxide, croscarmellose sodium, magnesium stearate, polyethylene glycol 3350, polyethylene glycol 6000, polyvinyl alcohol, talc, and titanium dioxide. In some embodiments, levetiracetam is administered as KEPPRA^{®} or an approved generic equivalent thereof.

In particular, an extended release form of levetiracetam, brivaracetam (BRIV) or selectracetam (SEL) is administered. In particular, an extended release tablet of levetiracetam comprises or consists of: (A) from about 30% w/w to about 85% w/w of levetiracetam; (B) from about 20% w/w to about 40% w/w of a water dispersible rate controlling polymer having a viscosity greater than 15 cps in a 2% w/w solution and selected from hydroxyethyl cellulose, hydrox- ypropyl cellulose, sodium alginate, carbomer, sodium carboxymethyl cellulose, xanthan gum, guar gum, locust bean gum, polyvinyl acetate, and polyvinyl alcohol, hydroxypropyl methylcellulose and mixtures thereof; (C) optionally a binder selected from polyvinyl pyrrolidone,hydroxypropyl cellulose, hydroxypropyl methylcellulose, methyl cellulose, poly- acryl amide, poly-N-vinyl amide, sodium carboxym- ethyl cellulose, polyethylene glycol, gelatin, polyethylene oxide, poly propylene glycol, tragacanth, alginic acid, and combinations thereof; (D) optionally one or more lubricants, anti adherents, or glidants, either alone or in combination; and (E) optionally a non-functional coating comprising hydrophilic film forming polymer, colorant and opacifying agent; wherein the tablet has the following dissolution profile in USP Apparatus 1 (basket) at 100 rpm in purified water at 37° C.: at time 2 hours the average of levetiracetam released is less than or equal to 44%, at time 4 hours the average of levetiracetam released is 35-75%, and at time 12 hours the average of levetiracetam released is greater than 75%.

In particular, the tablet provides extended therapeutically effective plasma levels over a twenty four hour period and further provides a peak blood plasma level of levetiracetam, brivaracetam (BRIV) or selectracetam (SEL) in from about eight to about sixteen hours.

In particular, the tablet consists of from about 50% to 80% levetiracetam, brivaracetam (BRIV) or selectracetam (SEL) by weight, about 20% to about 40% hydroxypropyl methylcellulose, by weight, and, optionally, from about 1% to about 5% polyvinyl pyrrolidone by weight.

In particular, the tablet consists of from about 61% to 73% levetiracetam, brivaracetam (BRIV) or selectracetam (SEL) by weight, about 25% to about 35% hydroxypropyl methylcellulose, by weight, and, optionally, from about 1.1% to about 1.5% polyvinyl pyrrolidone by weight.

In particular, the tablet is prepared by wet granulation, dry granulation or direct compression.

Certain extended release dosage forms of levetiracetam are marketed under various trade names, KEPPRA XR^{®}. KEPPRA XR^{®} tablets are film-coated extended-release tablets that contain 500 mg or 750 mg of levetiracetam. Inactive ingredients include colloidal anhydrous silica, hypromellose, magnesium stearate, polyethylene glycol 6000, polyvinyl alcohol-partially hydrolyzed, titanium dioxide (E171), Macrogol/PEG3350, and talc. In particular, levetiracetam is administered as KEPPRA XR^{®} or an approved generic equivalent thereof.

In particular, an injectable form of the synaptic vesicle 2a binding chemical entity, chosen from levetiracetam, brivaracetam (BRIV) or selectracetam (SEL)is administered intraperitoneally. In particular, an injectable form of the synaptic vesicle 2a binding chemical entity chosen from levetiracetam, brivaracetam (BRIV) or selectracetam (SEL) is administered intravenously.

### C. Sepsis

Sepsis is an infection with systemic inflammation. Normally, the immune and neuroendocrine systems control the local inflammatory process to eradicate pathogens. When this local control mechanism fails, systemic inflammation occurs, allowing the progression from infection to a diagnosis of sepsis, a diagnosis of severe sepsis, and a diagnosis of septic shock. As used herein, "sepsis" is characterized by the pressence of two or more of the following: increased or decreased temperature, increased or decreased leukocyte count, tachycardia, and rapid breathing. As used herein, "severe sepsis" is defined as sepsis with sepsis-induced organ dysfunction or tissue hypoperfusion (manifesting as hypotension, elevated lactate, or decreased urine output). As used herein, "septic shock" is sepsis with hypotension that persists after resuscitation with intravenous fluid. Thus, unless otherwise stated, "sepsis" encompasses clinical diagnoses of sepsis, severe sepsis, and septic shock.

### D. Prevention and/or Treatment

This invention as defined in the claims is directed to preventing an/or treating sepsis induced acute brain dysfunction (SiBD). The invention comprises administering a synaptic vesicle 2a binding chemical entity chosen from levetiracetam, brivaracetam (BRIV) or selectracetam (SEL), to a subject with sepsis. In particular, the subject has sepsis characterized by the presence of two or more of the following: increased or decreased temperature, increased or decreased leukocyte count, tachycardia, and rapid breathing, but does not yet have severe sepsis or septic shock. In particular, the subject has sever sepsis but does not yet have septic shock. In particular,the subject has septic shock.

This invention as defined in the claims is also directed to reducing sepsis mortality. The invention comprises administering a synaptic vesicle 2a binding chemical entity chosen from levetiracetam, brivaracetam (BRIV) or selectracetam (SEL), to a subject with sepsis. In particular, the subject has sepsis characterized by the presence of two or more of the following: increased or decreased temperature, increased or decreased leukocyte count, tachycardia, and rapid breathing, but does not yet have sever septis or septic shock. In particular, the subject has sever sepsis but does not yet have septic shock. In particular, the subject has septic shock.

In particular, the invention as defined in the claims comprise administering a synaptic vesicle 2a binding chemical entity chosen from levetiracetam, brivaracetam (BRIV) or selectracetam (SEL) to a subject with sepsis. In some embodiments, the synaptic vesicle 2a binding chemical entity is levetiracetam.

The synaptic vesicle 2a binding chemical entity chosen from levetiracetam, brivaracetam (BRIV) or selectracetam (SEL), may be administered orally, in a solid dosage form and/or a liquid dosage form. The synaptic vesicle 2a binding chemical entity chosen from levetiracetam, brivaracetam (BRIV) or selectracetam (SEL), may be administered parenteraly, such as subqutaneously, intravenously, or intramuscularly. In many cases in a subject with sepsis a parenteraly route of administration may be more appropriate because the condition may make oral administration difficult. In particular, during a course of prevention and/or treatment synaptic vesicle 2a binding chemical entity chosen from levetiracetam, brivaracetam (BRIV) or selectracetam (SEL), is initially administered to a subject parenteraly for a first period of time and then is administered orally for a second period of time.

In particular, half the daily dose of the synaptic vesicle 2a binding chemical entity chosen from levetiracetam, brivaracetam (BRIV) or selectracetam (SEL) is administered in two equaly doses every twelve hours. In particular, the daily dose is divided and administered in three equal doses every eight hours or in four equal doses every six hours. In particular, the daily dose of the synaptic vesicle 2a binding chemical entity chosen from levetiracetam, brivaracetam (BRIV) or selectracetam (SEL), is administered gradually over a four hour, six hour, eight hour, twelve hour, or twenty-four hour period, in particular the levetiracetam, brivaracetam (BRIV) or selectracetam (SEL) is administered parenteraly. Alternatively, when the synaptic vesicle 2a binding chemical entity chosen from levetiracetam, brivaracetam (BRIV) or selectracetam (SEL), is administered parenteraly it is administered as a single daily bolus or the daily dose is divided and administered as two, three, or four bolus doses per day.

In particular, the subject does not have epilepsy. In particular, the synaptic vesicle 2a binding chemical entity chosen from levetiracetam, brivaracetam (BRIV) or selectracetam (SEL), has not been administered to the subject prior to the subject developing sepsis. In particular, the subject has used the synaptic vesicle 2a binding chemical entity chosen from levetiracetam, brivaracetam (BRIV) or selectracetam (SEL), in the past but the synaptic vesicle 2a binding chemical entity chosen from levetiracetam, brivaracetam (BRIV) or selectracetam (SEL), has not been administered to the subject for at least one week, at least one month, at least three months, at least six months, or at least twelve months prior to the subject developing sepsis.

In particular, the synaptic vesicle 2a binding chemical entity chosen from levetiracetam, brivaracetam (BRIV) or selectracetam (SEL) is administered at a first dose for at least one day and then at a second dose for at least another day, wherein the first dose is higher than the second dose.

In some embodiments, the synaptic vesicle 2a binding chemical entity chosen from levetiracetam, brivaracetam (BRIV) or selectracetam (SEL), is administered for at least two days. In some embodiments the synaptic vesicle 2a binding chemical entity chosen from levetiracetam, brivaracetam (BRIV) or selectracetam (SEL), is administered for at least four days. In some embodiments the synaptic vesicle 2a binding chemical entity chosen from levetiracetam, brivaracetam (BRIV) or selectracetam (SEL) is administered for at least one week. In some embodiments the synaptic vesicle 2a binding chemical entitychosen from levetiracetam, brivaracetam (BRIV) or selectracetam (SEL) is administered for at least two weeks. In some embodiments the synaptic vesicle 2a binding chemical entity chosen from levetiracetam, brivaracetam (BRIV) or selectracetam (SEL), is administered for at least one month. In particular, the synaptic vesicle 2a binding chemical entity chosen from levetiracetam, brivaracetam (BRIV) or selectracetam (SEL), is administered to the subject until the subject does not have sepsis.

In particular, the synaptic vesicle 2a binding chemical entity chosen from levetiracetam, brivaracetam (BRIV) or selectracetam (SEL), is administered for at least one year. In particular, the synaptic vesicle 2a binding chemical entity chosen from levetiracetam, brivaracetam (BRIV) or selectracetam (SEL), is administered for the rest of the subject's life.

Administration of the synaptic vesicle 2a binding chemical entity chosen from levetiracetam, brivaracetam (BRIV) or selectracetam (SEL), according to use of the invention prevents and/or treats SiBD in a subject. In general, the prevention and/or treatment reduces at least one cognitive indicator and/or at least one physiological indicator of SiBD in the subject. Cognitive indicators include, by way of example, verbal fluency, attention, memory, working memory, praxies, also post traumtic stress disorders indicators, reviviscence syndrome, hypervigilance state, and nightmares. In some embodiments administration of levetiracetam to the subject reduces development of at least one cognitive indicator of SiBD in the subject.

In some embodiments the prevention and/or treatment reduces at least one sepsis indicator selected from Hospital Anxiety Depression Scale, Peritraumatic Distress Inventory, Peritraumatic Dissociative Experiences Questionnary, Impact of Event Scale-Revised.

In some embodiments administration of the synaptic vesicle 2a binding chemical entity chosen from levetiracetam, brivaracetam (BRIV) or selectracetam (SEL), to the subject reduces the risk of sepsis induced death in the subject. Reduction of risk of sepsis induced death occurs when levetiracetam is administered in a manner which has been demonstrated in a preclinical or clinical trial to reduce the rate of occurrence of sepsis induced death of subjects having a similar clinical diagnosis of sepsis.

In some embodiments administration of the synaptic vesicle 2a binding chemical entity chosen from levetiracetam, brivaracetam (BRIV) or selectracetam (SEL), to the subject reduces the risk of sepsis induced dementia in the subject. Reduction of risk of sepsis induced dementia occurs when the synaptic vesicle 2a binding chemical entity chosen from levetiracetam, brivaracetam (BRIV) or selectracetam (SEL), is administered in a manner which has been demonstrated in a preclinical or clinical trial to reduce the rate of occurrence of sepsis induced dementia in subjects having a similar clinical diagnosis of sepsis.

In some embodiments administration of the synaptic vesicle 2a binding chemical entity chosen from levetiracetam, brivaracetam (BRIV) or selectracetam (SEL), to the subject increases the cerebral level of IL1β in the subject. In some embodiments administration of the synaptic vesicle 2a binding chemical entity chosen from levetiracetam, brivaracetam (BRIV) or selectracetam (SEL), to the subject increases the cerebral level of IL4 in the subject. The cerebral level of these proteins is measured, for example, by obtaining a brain parenchyma sample from a subject and processing it as in the examples.

In some embodiments administration of the synaptic vesicle 2a binding chemical entity chosen from levetiracetam, brivaracetam (BRIV) or selectracetam (SEL), to the subject increases hippocampal expression of lba-1 in the subject. In some embodiments administration of the synaptic vesicle 2a binding chemical entity chosen from levetiracetam, brivaracetam (BRIV) or selectracetam (SEL), to the subject reduces cleavage of Caspase 3 in the brain of the subject.

Preventing and/or treating SiBD encompasses also preventing development of SiBD sequellae.

### E. Uses

Herein disclosed is the use of a synaptic vesicle 2a binding chemical entity chosen from levetiracetam, brivaracetam (BRIV) or selectracetam (SEL) for manufacturing a medicament intended to prevent and/or treat SiBD in a subject in need thereof. In particular, the synaptic vesicle 2a binding chemical entity is levetiracetam.

Herein disclosed is the use of a synaptic vesicle 2a binding chemical entity chosen from levetiracetam, brivaracetam (BRIV) or selectracetam (SEL) for manufacturing a medicament intended to reduce sepsis mortality. In particular, the synaptic vesicle 2a binding chemical entity is levetiracetam.

### EXAMPLES

### Material and Methods

**Experimental plan:** To reduce subjective bias, every animal was randomized and every analysis was performed after attribution of a randomization number, revealed at the end of experiments.

**Cecal ligature and puncture:** Cecal ligature and puncture on mice were performed under general anesthesia. Briefly, C57Bl/6 mice were anesthetized with an intraperitoneal injection of Xylazine (20mg/Kg, Rompun 2%, Bayer, Leverkusen, Germany), and Ketamine (100mg/Kg, Imalgene, Mérial, Lyon, France) in 100 µl of saline solution at 0.9%. After anesthethic administration, mice were placed in a heated cage or surgical table upon awakening to maintain a body temperature at 37°C. Absence of reaction to leg and tail pinch were checked before incision of abdominal wall and peritoneum. Caecum was found with wet coton swabs and exposed before loose ligation with Mersilk 4.0 (Ethicon, Cincinatti, USA), 2 transfixiant punctures were realized avoiding blood vessels with a 21G needle. Feces were expressed between coton swabs before suturing abdominal muscles and peritoneum in 2 separate plans.

**Randomization** : After closing abdominal wall, animals were randomized through random number generation (random.org) to receive either levetiracetam or a placebo composed of NaCl 0,9%. Analgesia and rehydratation of mice were performed with buprenorphine (0.1mg/Kg, Buprecare, Axience, Pantin, France) every 12 hours until complete recovery. They received levetiracetam just after and 6, 12, 24 and 36 hours after sepsis induction.
Clinical scoring: Sepsis severity was evaluated twice a day until day 4, or normalization of « sepsis score », a previously described score that ranges from 1 to 12. This score evaluates markers of local (abdominal spasm) and systemic reaction to sepsis (rectal temperature, fur erection, abnormal breathing), behavioural alterations (feces cleaning, presence of periocular dried eyedrop, incomplete palpebral opening, spontaneous activity in the cage, the escape attempt after tail grabbing) and signs of muscular weakness (grip strength, body tone, difficulty to walk). Each item was scored 0 or 1, with a total score rang from 0 (normal) to 12 (highly severe). For ethical reason, mice with a sepsis score ≥8 and a temperature ≤34.5°C were euthanized as they were considered to almost certainly die within the 12 hours.

**Sacrifice:** Mice were sacrified after deep anesthesia, blood was collected in the heart in heparinized syringes (Heparine Choay 50 000 UI/mL, Sanofi, France). Brains were removed using forceps after skin incision and section of the skull with small scissors, and further processed depending on the different applications.

**Cytokine multiplex:** Brain samples were weighed and unfrosted in 100 µl of extraction buffer (PBS 1x, complete protease inhibitor, sigma-aldrich, triton 0.1%) processed in 2 ml Lysing Matrix A tubes with FastPrep-24 (MP biomedicals, Germany). Tubes were centrifuged at 20000g at 4°c and supernatant was collected and protein concentration was determined with light absorbance at 280nm using a Nanodrop Lite Spectrophotometer (Thermo Scientific). Sera were used undiluted. Cytokines were measured using a Bio-Plex System and Mouse Cytokine Standard 23-Plex Group 1 kit following manufacturer's recommendations. Raw datas were analyzed by dividing the concentration of cytokine with protein concentration in lyzate and weight of sample measured on precision balance. Then every cytokine concentrations are presented as a ratio compared to the untreated sepsis group.

**RNA Purification:** Total brains or mircodissected brain under binoccular magnifier were collected in liquid nitrogen and stored at -80°C before processing. Tissues were unfrosted in 1ml of Qiazol (Qiagen, Nederland) and shredded uzing a TissueLyzer II and metallic beads (Qiagen, Nederland). Two hundred microlitters of chloroform (Merck, Darmstädt, Deutschland) were added before spinning for 15' at 4°C - 20 000rpm. The upper aquous phase was collected and purified using RNEasy Minikit with a DNAse step (Qiagen, Nederland) to remove any remaining genomic DNA. Purified RNA were quantified and a reverse transcription on 500 ng of RNA using SuperScript^{®} II Reverse Transcriptase (Life Technologies) was realized. Quantitative real time polymerase chain reaction was performed on 10 ng cDNA with SYBR^{®} Select Master Mix (Roche, Franche) following manufacturer's recommandation on a StepOne^{™} Real-Time PCR System (Life Technologies) and results were analyzed on StepOne softwares. Triplicates with a Cycle Threshold standard deviation <0.2 were considered for further anlyzes. ΔΔCT method was used to quantify fold increases between animals from different groups to control group mean. The following primers were used.

| | | | | |
|---|---|---|---|---|
| *CD32* | Cluster of differentiation 32 (CD32) | CTG GAA GAA GCT GCC AAA AC (SEQ IS NO : 1) | CCA ATG CCA AGG GAG ACT AA(SEQ IS NO : 2) | NM_010187.2 |
| *CD86* | Cluster of differentiation 86 (CD86) | GAG CGG GAT AGT AAC GCT GA (SEQ IS NO : 3) | GGC TCT CAC TGC CTT CAC TC (SEQ IS NO : 4) | NM_019388.3 |
| *Ptgs2* | Cyclooxygena se-2 (Cox-2) | TCA TTC ACC AGA CAG ATT GCT (SEQ IS NO : 5) | AAG CGT TTG CGG TAC TCA TT (SEQ IS NO : 6) | NM_011198.3 |
| *CD16* | Cluster of differentiation 16 (CD16) | TAC ACA GCA CCA GTC CAA GC SEQ IS NO : 7) | AGA AAT AAA GGC CCG TGT CC SEQ IS NO : 8) | NM_010188.5 |
| *CD206* | Cluster of differentiation 206 (CD206) | CTT CGG GCC TTT GGA ATA AT (SEQ IS NO : 9) | TAG AAG AGC CCT TGG GTT GA (SEQ IS NO : 10) | NM_008625.2 |
| *Arg1* | Arginase-1 (Arg1) | GTG AAG AAC CCA CGG TCT GT (SEQ IS NO : 11) | GCC AGA GAT GCT TCC AAC TG (SEQ IS NO : 12) | NM_007482.3 |
| *Lgals3* | Galectin-3 (Gal-3) | GAT CAC AAT CAT GGG CAC AG (SEQ IS NO : 13) | ATT GAA GCG GGG GTT AAA GT (SEQ IS NO : 14) | NM_010705.3 |
| *Igf1* | Insulin like growth factor 1 (IGF-1) | TGG ATG CTC TTC AGT TCG TG (SEQ IS NO : 15) | GCA ACA CTC ATC CAC AAT GC (SEQ IS NO : 16) | NM_010512.4 |
| *Ccr2* | C-C chemokine receptor type 2 (CCR2) | GCC AGG ACA GTT ACC TTT GG (SEQ IS NO : 17) | TTC CTG GTA GAG AGG CAA ACA (SEQ IS NO : 18) | NM_009915.2 |
| *Tgfb* | Transforming growth factor β (TGFβ) | CTT TTG ACG TCA CTG GAG TTG (SEQ IS NO : 19) | CAG TGA GCG CTG AAT CGA A (SEQ IS NO : 20) | NM_011577.1 |
| *Cxc3cr1* | CX3C chemokine receptor 1 (CXC3CR1) | AAG TTC CCT TCC CAT CTG CT (SEQ IS NO : 21) | GGA CAG GAA GAT GGT TCC AA (SEQ IS NO : 22) | NM_009987.3 |
| *Sphk1* | Sphingosine kinase 1 (Sphk1) | TCC AGA AAC CCC TGT GTA GC (SEQ IS NO : 23) | CAG CAG TGT GCA GTT GAT GA (SEQ IS NO : 24) | NM_001172475. 1 |
| *Sphk2* | Sphingosine kinase 2 (Sphk2) | CAG GGG TTA AGC CTG AGT GA (SEQ IS NO : 25) | AGT CTG GCC GAT CAA GGA G (SEQ IS NO : 26) | NM_001172561. 1 |
| *Il1rn* | Interleukin 1 receptor antagonist (IL-1Rn) | TTG TGC CAA GTC TGG AGA TG (SEQ IS NO : 27) | TTC TCA GAG CGG ATG AAG GT (SEQ IS NO : 28) | NM_031167.5 |
| *Il4ra* | Interleukin 4 receptor alpha (IL-4Rα) | GGA TAA GCA GAC CCG AAG C (SEQ IS NO : 29) | ACT CTG GAG AGA CTT GGT TGG (SEQ IS NO : 30) | NM_001008700. 3 |
| Vim | Vimentine | cggaaagtggaatccttgca (SEQ IS NO: 31) | cacatcgatctggacatgctgt (SEQ IS NO: 32) | NM_011701,4 |
| Tnf | Tumor necrosis factor alpha | GACCCTCACACTCAGATCATCT TCT (SEQ IS NO : 33) | CCTCCACTTGGTGGTTTGCT (SEQ IS NO : 34) | NM_001278601. 1 |
| Il1b | Interleukine 1 beta | CTGGTGTGTGCAGTTCCCATT A (SEQ IS NO : 35) | CCGACAGCACGAGGCTTT (SEQ IS NO : 36) | NM_008361,3 |
| Il1a | IL-1 alpha | CAAACTGATGAAGCTCGTCA (SEQ IS NO : 37) | TCTCCTTGAGCGCTCACGAA (SEQ IS NO : 38) | NM_010554,4 |
| GFAP | GFAP | AAGCCAAGCACGAAGCTAAC (SEQ IS NO : 39) | ATTTGCCGCTCTAGGGACTC (SEQ IS NO : 40) | NM_010277,3 |
| IL4 | IL-4 | CATCGGCATTTTGAACGAGGT CA (SEQ IS NO : 41) | CTTATCGATGAATCAGGCAT CG (SEQ IS NO : 42) | NM_021283,2 |
| IL10 | IL-10 | CCAGTTTTACCTGGTAGAAGT GATG (SEQ IS NO : 43) | TGTCTAGGTCCTGGAGTCCA GCAGACTC (SEQ IS NO : 44) | NM_010548,2 |
| Ccl2 | chemokine (C-C motif) ligand 2 or MCP-1 | CCCAATGAGTAGGCTGGAGA (SEQ IS NO : 45) | TCTGGACCCATTCCTTCTTG (SEQ IS NO : 46) | NM_011333,3 |
| GAPDH | GAPDH | TGATGACATCAAGAAGGTGGT GAAG (SEQ IS NO : 47) | TCCTTGGAGGCCATGTGGG CCAT (SEQ IS NO : 48) | NM_001289726, 1 |
| Actb | beta actin | CTAAGGCCAACCGTGAAAAG (SEQ IS NO : 49) | ACCAGAGGCATACAGGGAC A (SEQ IS NO : 50) | NM_007393,3 |
| Rn18s | ARN Ribosomal 18s | CGG ACA GGA TTG ACA GAT TG (SEQ IS NO : 51) | CAA ATC GCT CCA CCA ACT AA (SEQ IS NO : 52) | |
| IL6 | Interleukine 6 | CAAAGCCAGAGTCCTTCAGAG (SEQ ID NO: 57) | TGGTCCTTAGCCACTCCTTC (SEQ ID NO: 58) | |

**Comportemental tests:** Every comportemental tests were started 15 days after sepsis induction. The experimentator was blinded to the experimental group.

**Novel Object Recognition Test:** Tests comprise three phases: habituation, familiarization and test phase. During the first 20 minutes animals were placed in a transparent arena under a 100 lux light that had three compartments separated with one-holed plexiglass walls. Two objects were used (a plastic power plug and a brass bolt). During the next 10 minutes two similar objects (A,A) were placed in the lateral compartments. We considered that mice were exploring the objects when they were climbing or explore it with their nose. Immediately after mice were replaced in the center of arena and two new objects (A,B) were placed in the compartments. The ratio of time spent exploring objects A and ratio of time exploring object A/B were compared.

**Odor Habituation:** In a transparent cage with perforated aluminium soil under a 100 lux light, different odors were presented on a paper filter lying on parafilm. The odor A was presented during three 2-minutes trial with a 2 minute wash out period. A time ratio between the third and first trial of 0.6 was then considered to be a success of memorisation. A new odor B was presented instead of A in the 4th trial to the animal. Absence of odor defect was assessed by an increase of time spent smelling the new odor during the fourth trial.

**Fear conditioning.** Mice underwent the classical fear conditioning test with a cued stimulus (auditory tone) and an uncued stimulus (electric shock). During two consecutive conditioning trials, 2 minutes resting followed by a 48s tone (440KHz tone, 60 db) and 5 electrical footshocks of 2 joules during 2 seconds. Twenty four hours after conditioning, mice underwent the fear memory recall after exposition to a contextual stimulus (shock cage in the same configuration without auditory tone) or an auditory stimulus (Auditory tone in a different cage with a vanilla artificial odor). Freezing was evaluated for 2 seconds every 8 seconds and was defined as the absence of movement of the mouse with tail erection. Percentage of 10 seconds period freezing was reported to the total amount of freezing.

**Morris Water Maze** was realized as described by Vorhees et al. Nature Protocols 2006. It consisted in a phase of spatial learning and a probe trial. During the first phase the animal learns spatial position of a platform (11 cm diameter) hidden in a swimming pool (120 cm diameter) with opaque water relatively to cues placed on the water tank and in the experimental room. The animal is released in a random quadrant 4 times per day during 7 days. The distance (cm) before reaching the platform, the latency (s) and the proportion of time spent in each quadrant is measured. After each day the learning curve of mice reaching the platform is drawn. If this curve reached a plateau, the probe trial is realized the next day. Probe trial consists in a recall of the spatial reference memory.

**Open Field Mice were placed 45 minutes before being held in the experimental room.** Single mice were then placed in a squared 40 cm box in an experimental room and captured with a camera during 10 minutes. Mice were tracked using Ethovision XT and their tail, nose and barycenter was marked. The total length walked, the ratio of time spent moving/non moving, the maximum velocity and the mean distance from barycenter to arena's center were recorded. Datas are presented as Mean±SEM.

**Splash test:** Mice were placed in a new cage 45 minutes before testing in the experimental room. They were then held onto a grid and 200 µl of sucrose 10% solution was spray on their back's fur. They were then placed in the home cage for 5 minutes. The latency before the first time mice groomed, the number of cleaning sessions and the total amount of time spent cleaning during the 5 minute test were measured. Mice were then placed back in their cage. Datas are presented as Mean±SEM.

**Elevated plus maze test:** Mice home cage was placed in the experimental room 45 minutes prior any handling. Mice were placed in cross shaped 40 cm maze consisting in 2 open arms without walls and 2 closed arms with 30 cm opaque walls. Mice were tracked with ethovision XT during 5 minutes trials. The maximum velocity, total distance walked, number of entries in an open arm, and amount of time spent in an opened arm or in a closed arm were recorded. Datas are presented as Mean±SEM.

**Immunohistochemistry:** Mice were perfused through left ventricule with paraformaldehyde 4% and the collected brains with small forceps. Brain were fixed overnight with PFA 4% and then cut in 100µm sagittal slices with a vibratome VT1200S (Leica, Germany). Ionized calcium binding adaptor type 1 immunohistochemistry. Brain sections were washed with PBS and a specific binding sites were saturated with a bovine serum albumine 3% in PBS solution for 4 hours. Then primary antibody (Rabbit, Wako, 40ng/µl) was applied for 24 hours at 4°C under constant agitation. Cyanine 3 secondary antibody (Donkey anti-Rabbit) and Hoechst 33342 (1/4000) was then applied overnight at 4°C with constant agitation.

**GFAP Immunohistochemistry:** Slices were saturated and incubated overnight in PBS-BSA 3% buffer with triton X100 solution. Primary antibody against glial fibrillary acidic protein GFAP (chicken ABCAM, ab4674) was applied for 24 hours with constant agitation at 4°C.

**Imaging** of samples was realized in a medium throughput spinning disk confocal microscope (CV1000) on 5x5 mozaics reconstructed with an overlap of 50% to avoid signal intensity lowering on the edge of tiles. Stitching was realized using a homemade macro with the Grid/collection plugin for Fiji channel by channel and then manually realligned if necessary. Automated analysis of microglial cell was realized on Iba1 staining. Automatic local contrast thresholding using Phanshalkar algorithm with a radius of 2 µm was performed for microglia channel, and with a radius of 12 µm for nuclei channel. Microglial cells were identified using a fixed empirical threshold for percentage of perinuclear area stained by Iba-1 using thresholded image. Only individual microglial cells obtained after segmentation with a tridimensionnal watershed algorithm were furthered considered for analysis. Morphometric study of microglial cells were performed using mcib3d plugin for FIJI. Concentric enveloppes were computed starting 1 µm after nuclear enveloppe to analyze the number of microglial process crossing this sphere and reproduce a conformationnal tridimensional analysis on microglial cells.

Individual and summarized data were then analyzed in R statistics. Area under the curve of number of microglial processes. When covariant variables were identified, the most relevant was considered.

### Example 1: Levetiracetam Reduces Death and Cognitive and Behavioural Impairments After Sepsis

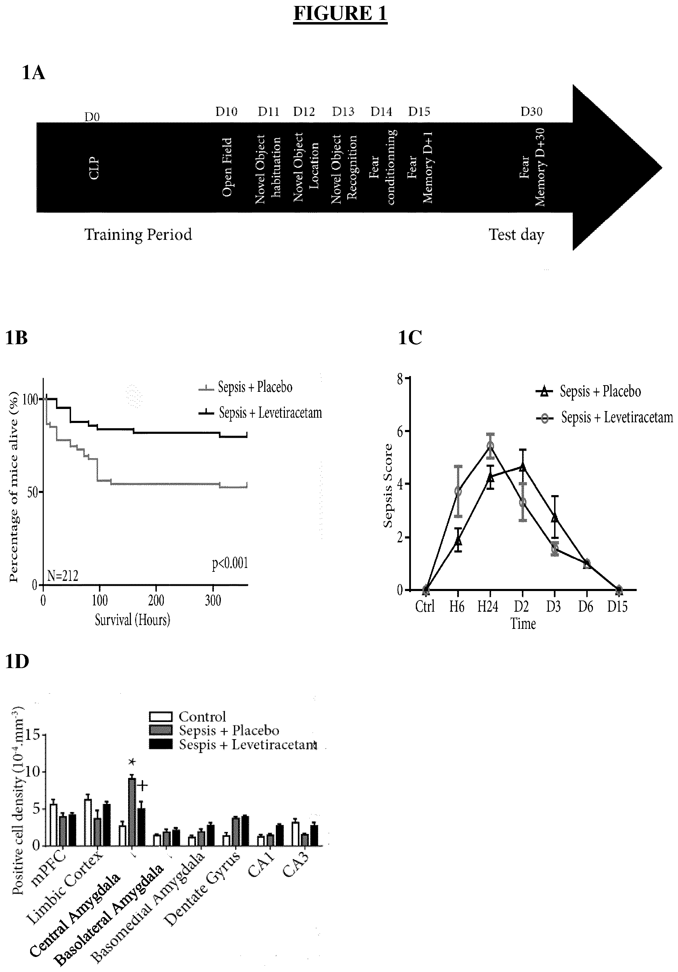

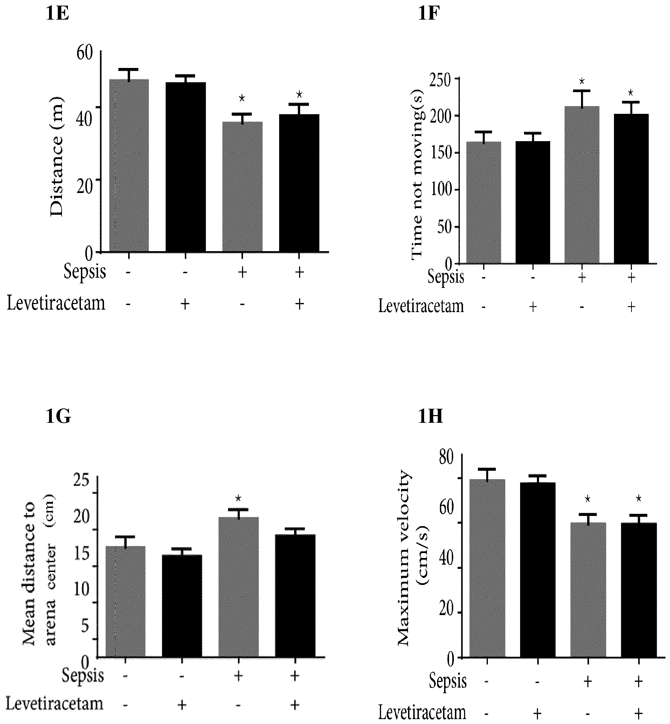

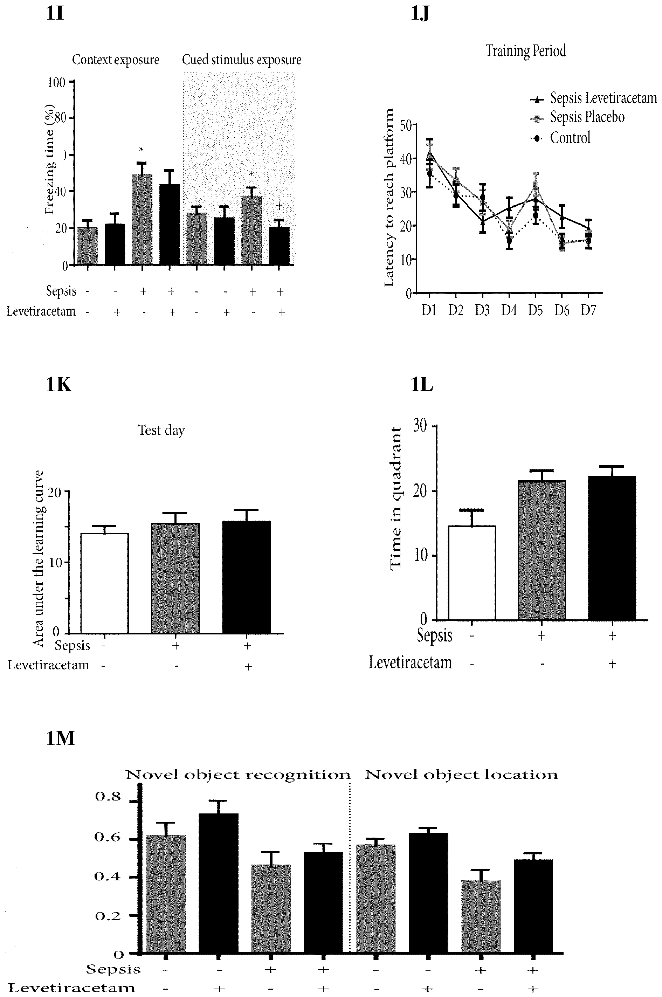

**Figure 1A****.** Schematic of experiments performed in CLP mice randomly treated with levetiracetam or placebo.

**Figure 1B****.** Survival of mice who underwent CLP and received intraperitoneously saline (100µl, light grey plein curve) or levetiracetam (300mg/Kg in100µl, black curve). Mice were followed every 12 hours. Survival is shown as a kaplan-meyer curve with a log rank test. Mice were considered dead after they reached limit points and censured if they were sacrificed for experiments. The administration of Levetiracetam was associated with a dramatic reduction in mortality within the first four days : survival rate increased from 53 % in placebo treated mice to 81% in levetiracetam treated mice (n=212, p<0.001).

**Figure 1C****.** Sepsis score was evaluated every 12 hours (general state criteria, temperature, fur erection, respiratory rate, sickness behaviour, spontaneous activity in the home cage, lacrimation, palpebral closure, feces toilet, locomotor criteria, grip strength, difficulties walking, local reaction, abdominal wall contraction, axial body tone). Datas are presented as mean±SEM. Sepsis scores did not differ between CLP mice treated with levetiracetam and placebo.

**Figure 1D****-** density of c-fos positive neurons in the different brain areas after sepsis in mice treated with levetiracetam or placebo. c-fos expression pattern was analysed in 100µm thick sections of amygdala revealed with AEC. Datas are presented as mean±SEM. mPFC medial prefrontal cortex prelimbic and cingular area, CA1 *Cornu Ammonis* area 1 CA3 *Cornu Ammonis* area 3 CLP. Cecal Ligature and puncture. Animals who underwent CLP presented a higher density of neurons expressing c-fos in their nuclei 6 hours after sepsis induction in the central nuclei of the amygdala (8 *vs.* 3×10⁻⁴.mm⁻³, p<0.05). The expression of c-Fos peaked at H6 and decreased to normal values 24 hours after sepsis. C-fos expression pattern in the different brain areas is modified by levetiracetam administration. Levetiracetam lowered specifically c-fos expression in the central amygdala (CeA) at H6 (p<0.05).

**Figure** E, F, G. Openfield tests 10 days after sepsis in mice receiving levetiracetam or saline. Datas are presented as mean±SEM. Sepsis presented a persisting effect on locomotor functions : sepsis lowered the distance traveled (E, p<0,05), increased the time spent not moving (F, p<0,05) and the mean distance to center (G, p<0,05) compared to control mice. Openfield tests showed that levetiracetam did not influenced sepsis-associated lowering of distance traveled (E) nor time spent not moving (F) but decreased the mean distance to center in septic mice (p=0.043, Mann-Whitney, G). This shows that levetiracetam reduces post-septic anxiety-like behavior.

**Figures H-I**- Fear conditioning tests showed that motor response to electric shock did not differ between groups during conditioning period **(H)** and that auditory freezing time in response was lower in levetiracetam treated than placebo treated CLP mice (p=0,024, Mann-Whitney N= 11, N=9,N=10, N=14, **I).** This shows that Levetiracetam reduces post-septic fear memorization.

**Figures J-M**. Morris water Maze test (MWM) and Novel Object Location/Recognition tests. **J-K.** Control mice showed a trend towards faster learning of how to locate the platform in the morris water maze as suggested by the lower AUC of the learning curve. Levetiracetam did not present an effect on this hippocampal function. **L.** During the test day, mice spent the same amount of time in the platform quadrant. **M.** The novel object location and novel object recognition tests showed that control mice recognized the novel object location (ratio of time spent in seconds on novel object / time spent on the familiar object >65%) but septic mice spent the same amount of time on novel or familiar object whether they were treated or not.

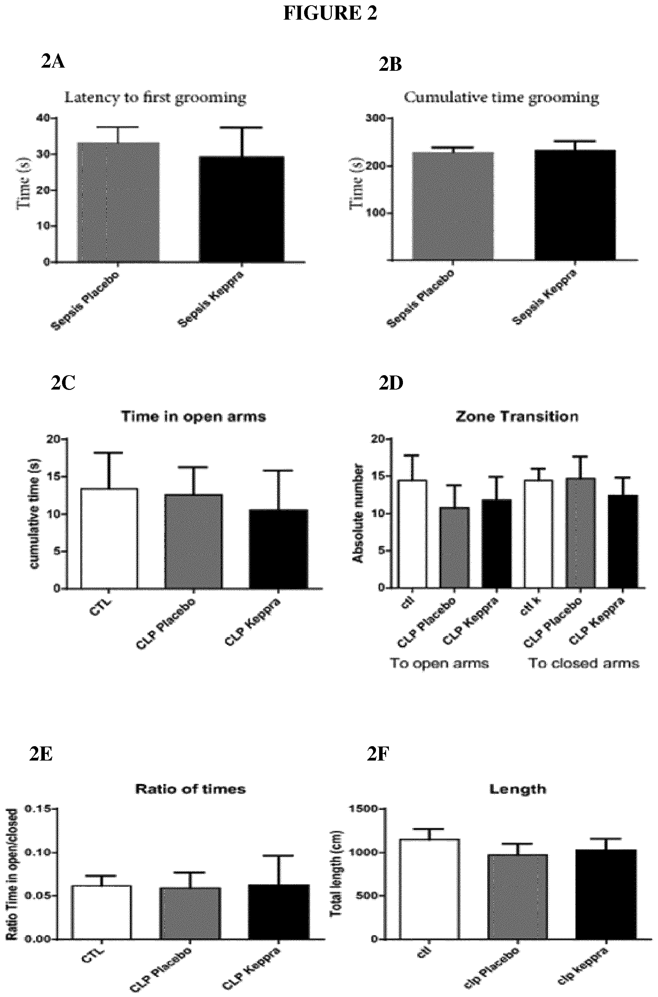

**Figure 2****:** Innate anxiety behavior and depressive phenotype was investigated with the elevated plus maze and splash test. No difference in the innate anxiety and depressive phenotype was observed between levetiracetam and placebo treated mice that underwent CLP.

### Example 2: Levetiracetam effect on inflammation and neuroinflammation

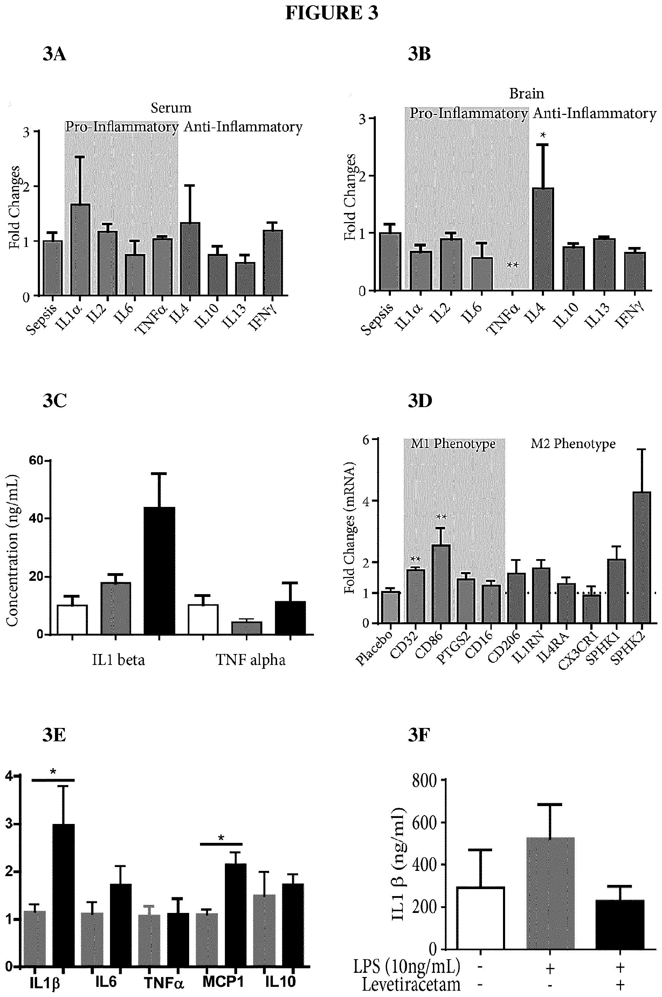

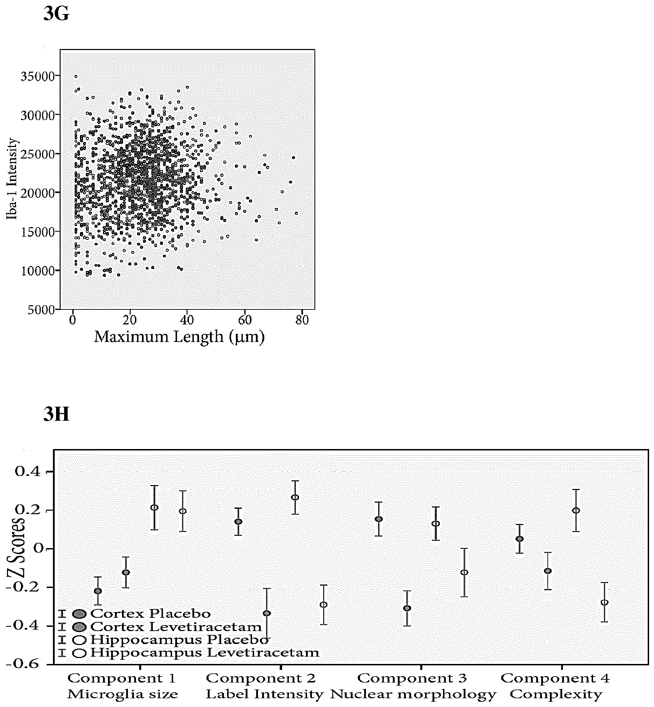

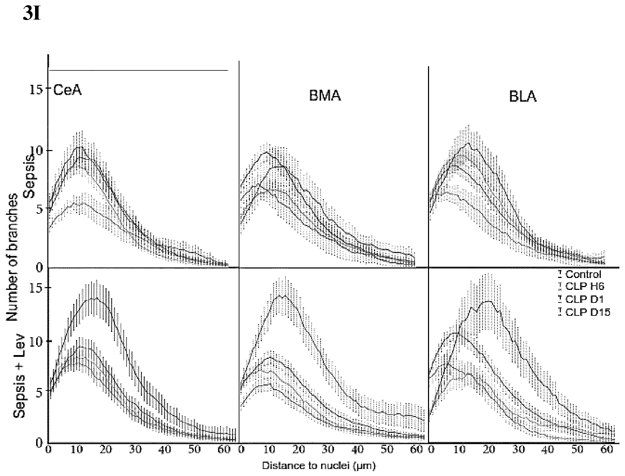

**Figure 3** **(A,B)-** Multiplex dosage of pro- and anti-inflammatory cytokine levels in Serum **(A)** and brain parenchyma **(B),** 24 hours after sepsis induction in mice treated or not with Levetiracetam (mean fold increased ±SEM compared to control group set at 1, N=11 and 12). No significant effect of levetiracetam treatment on cytokine concentration in plasma after sepsis was observed. In the brain parenchyma macerated in saline, significant changes in cytokine concentration were observed in septic mice receiving levetiracetam versus septic mice receiving saline (p<0,001, ANOVA). TNF alpha could not be detected in the sepsis group treated with levetiracetam, also IL4 concentration was increased in septic mice receiving levetiracetam versus septic mice receiving saline. Whereas levetiracetam did not present an effect on systemic inflammation, a modulation of neuroinflammation was observed.

**Figure 3C****-** An ELISA assay was performed on brain parenchyma to detect lower changes in cytokine concentration. No difference in TNF alpha expression during sepsis or with treatment with levetiracetam was shown whereas IL1 beta was increased with levetiracetam treatment (p<0,05) during sepsis.

**Figure 3** **(D,E)-** mRNA expression of pro-inflammatory (M1 phenotype) and anti-inflammatory (M2 phenotype) microglial markers **(D)** and cytokines **(E)** in CD11b positive cells (microglial cells), 24 hours after sepsis in mice receiving levetiracetam or saline. Datas are presented as mean±SEM compared to septic placebo group set at 1. CD32 and CD86 transcription was increased in the sepsis group receiving levetiracetam vs. Placebo (Mean±SEM **Panel D).** Anti inflammatory markers were not increased neither genes promoting anti-inflammatory phenotypes **(Panel D).** mRNA from microglial cells showed also an increased transcription of proinflammatory genes (Il1 beta and MCP 1 increase, Mean±SEM **Panel E).** Microglial cell sorting in whole brain of septic mice receiving levetiracetam showed a surprising result. Microglial cells were polarized towards a pro-inflammatory (M1) phenotype.

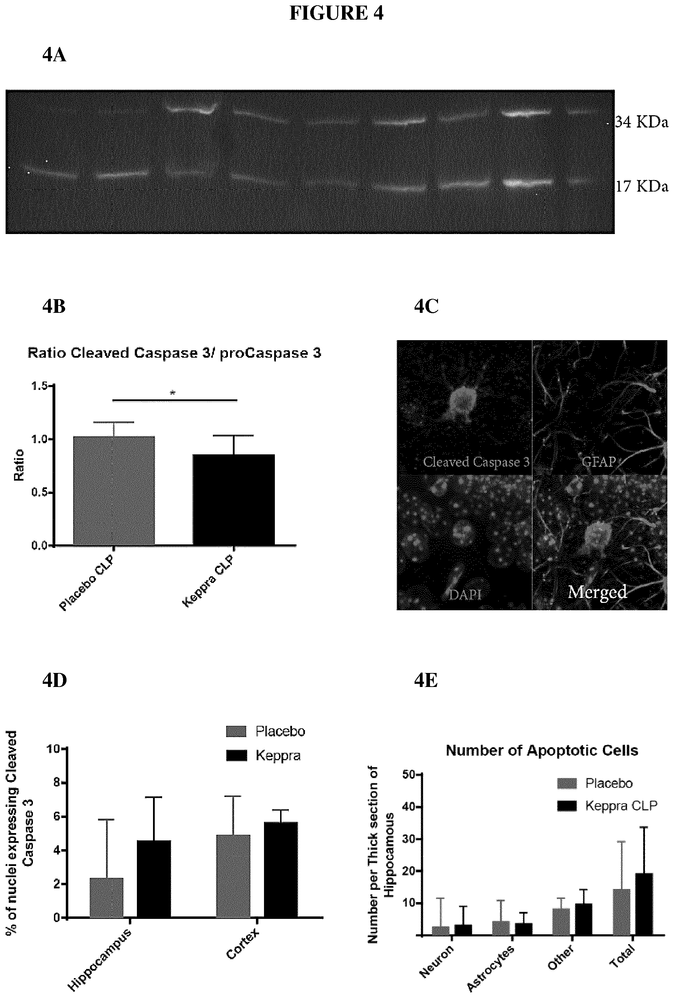

**Figure 3F****.** IL1 beta production in primary microglial cell culture exposed to LPS with or without levetiracetam (NS). The effect of Levetiracetam on neuroinflammation modulation (Figure 4B) was also observable on IL1 in primary microglial cell culture after stimulation by LPS.

**Figure 3** **(G, H, I).** Morphological analysis of microglial cell morphology changes in the brain during the first 15 days after sepsis in mice treated or not with levetiracetam.

**G.** Morphological study of microglial cells in the brain based on Ionic Binding adapter 1 protein, involved in calcium signaling that marks precisely cytoplasm of microglial cells. No correlation was found between intensity of Iba-1 labelling (in arbitrary units) and the longest microglial branche (in µm).

**H.** Principal component analysis of the 107 morphological criterion measured was performed using homemade morphological analysis algorithm to define 4 main components related to sample labeling, microglial size, nuclear morphology and qualitative analysis of microglial cell body. These 4 different components are different when levetiracetam is administered to the mice pointed a more complex and with a biggest volume microglial cell in the serum group suggesting an activated phenotype of microglial cells in the levetiracetam group.

**I.** Tridimensionnal analysis of microglial cell branching on Iba-1 staining after sepsis during the first 15 days after sepsis in mice treated or not with levetiracetam. During sepsis, number of cell branches was decreased in the Central Amygdalia (CeA) and the Basolateral Amygdalia (BlA) at 6 hours and were normal after 15 days. Levetiracetam administration during sepsis decreased microglial cell morphological changes at 6 hours after sepsis but increased dramatically the number of cell processes 15 days after sepsis. Tridimensionnal analysis of microglial cell branching on Iba-1 staining after sepsis showed a region speficic microglial cell activation in the CeA and the BlA, which could be reduced by levetiracetam administration.

Levetiracetam might promote a more inflammatory phenotype in whole brain but might dampen microglial activation in specific region, notably the CeA

### Example 3: Analysis of Astrocytes and Cell Death

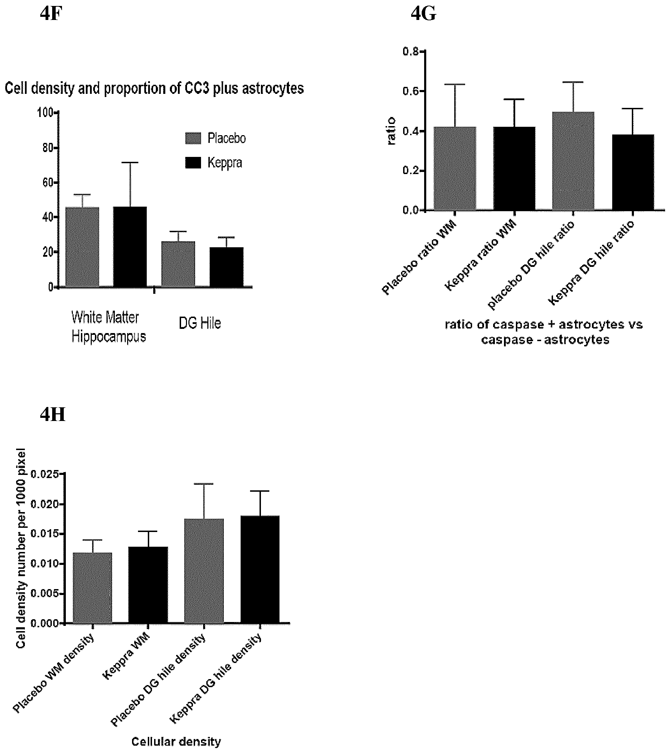

**Figure 4****:** Astrocytic reaction and cell death analysis after sepsis in Levetiracetam (Keppra) treated mice and saline treated mice (Placebo). **A-** Western Blot from cleaved caspase 3 from macerated brain parenchyma. **B**-Mean ± SEM of ratios between intensity of cleaved caspase 3 vs. procaspase 3. **C**-Caspase 3 immunohistochemistry showed intense labelling in few cells cytoplasm (late apoptosis stage) and light labelling in some nuclei (early apoptosis stage). D-G Cell count for late and early stage of apoptosis. WM Whitte matter, DG, Dentate Gyrus, CC3 Cleaved Caspase 3. These results show that levetiracetam reduces cleavage of Caspase 3 in the brain of septic mice. These results show a lower global cleavage of caspase 3 in whole brain without modification in the number of apoptotic cell which is a very dynamic process and less sensitive endpoint to assess the prevention of apoptosis through a lower caspase 3 cleavage.

### Example 4 : Effect of Levetiracetam on activated microglial cell cultures

### Material and methods

### Cell culture

Primary mixed glial cell cultures were obtain from dissected cortices of postnatal day (P) 0-1 C57/Bl6 mice. An equal number of male and female were included in each culture. Cortices were dissected in 0.1 M PBS with 6% glucose and 2% penicillin-streptomycin (Gibco, Cergy Pontoise, France) meninges were removed. Cortices were chopped into small pieces and mechanically dissociated in pipettes, then diluted in pre-cooled low glucose Dulbecco's modified Eagle's minimum essential medium (DMEM, 31885, Gibco) with 10% foetal bovine serum (FBS, Gibco) and 0.01% penicillin streptomycin. Microglia were isolated from primary mixed glial cultures on day in vitro 7 using a reciprocating shaker (20 min at room temperature) and rinsed in the culture medium using a 10 mL pipette. Microglial cells were pelleted via centrifugation (300g × 10 min) and resuspended in DMEM supplemented with 10% FBS at a concentration of 4 × 10^5 cells/mL in 6-well culture plates. Based on previous reports, the day after plating, microglia were treated with PBS (vehicle; 10 µl/ml of culture media), LPS at 1 ng/mL and 10 ng/mL, and levetiracetam (UCB, Belgium, Brussel) at two concentrations (10 and 100 micrograms/mL). Twenty four hours after LPS exposure supernatant (conditioned media) was collected and stored at -80 °C until analysis of cytokine/chemokine levels and cells were harvested in RNA Later (RLT buffer, Qiagen, Courtaboeuf, France)) and RNA extracted for gene expression analysis.

### RNA purification and quantification

Total RNA from primary microglial cell cultures was extracted with the RNeasy mini kit according to the manufacturer's instructions (Qiagen, Courtaboeuf, France). The quality and concentration of total RNA were assessed with the Nanodrop^{™} apparatus (Thermoscientific, Wilmington, DE, USA). Reverse transcription was performed on one microgram of total RNA using the iScript^{™} cDNA synthesis kit (Bio-Rad, Mames-la-Coquette, France). Duplicate quantification in real time PCR was performed using SYBR Green Supermix (Bio-Rad) for 40 cycles with a program including 2 steps (5 s of denaturation at 96 °C and 10 s of annealing at 60 °C). Single peak in melting curves assessed primer specificity. Primers were retrieved from previously published sequences (Chhor et al., Brain Behav. Immun., 2015, Volume 32, August 2013, Pages 70-85). Primer sequences for Cluster of differentiation 32 (CD82), Cluster of differentiation 86 (CD86), Cyclooxygenase 2 (Ptsg2 or cox-2), Cluster of differentiation 16 (CD16), Cluster of differentiation 206 (CD206), Arginase-1 (Arg1), Galectin-3 (Lgals3), Insulin Growth Factor 1 (IGF-1), Interleukin 1 receptor antagonist (IL-1Rn) and Interleukin 4 receptor alpha (ILA-Rα), Tumor necrosis factor alpha (TNFα), and Interleukin 1 beta (Il1b) genes were as described in Table from Material and Methods section of preceding examples. Primers for Glyceraldehyde 3-phosphate dehydrogenase (*GAPDH*) gene were: sense primer: GGC CTT CCG TGT TCC TAC (SEQ ID NO: 53) and anti-sense primer : TGT CAT CAT ATC TGG CAG GTT (SEQ ID NO: 54). Primers for Inducible nitric oxide synthase (iNOS) gene were: sense primer : CCC TTC AAT GGT TGG TAC ATG G (SEQ ID NO: 55) and anti-sense primer ACA TTG ATC TCC GTG ACA GCC (SEQ ID NO: 56). Primers for Interleukin 6 (IL6) gene were: sense primer : CAAAGCCAGAGTCCTTCAGAG (SEQ ID NO: 57) and anti-sense primer : TGGTCCTTAGCCACTCCTTC (SEQ ID NO: 58). RNA quantity was analyzed with 2e^-ΔΔCT using the CT of genes of interest relative to expression of the reference gene, Glyceraldehyde 3-phosphate dehydrogenase (*GAPDH*).

### Results

### Cytokine transcription

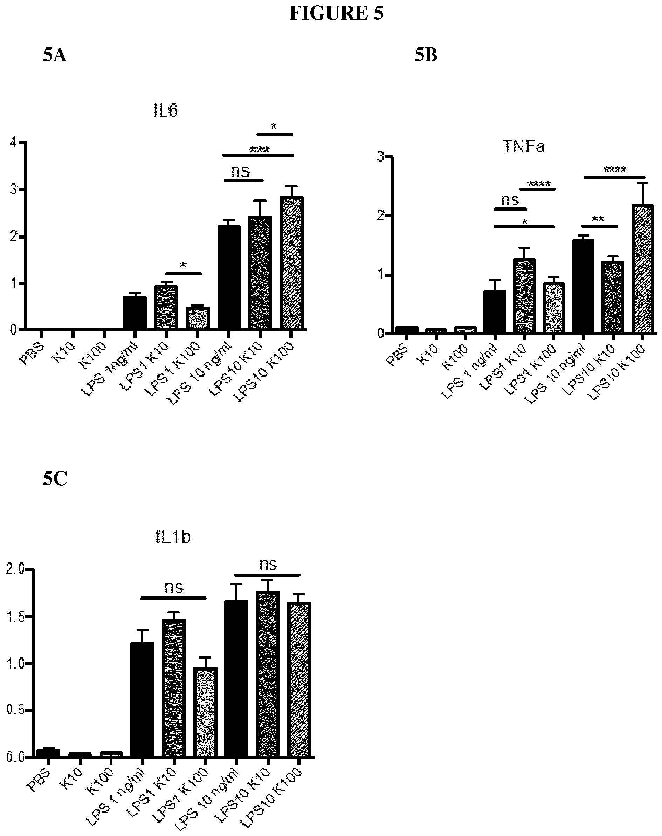

LPS increased the expession of IL6, TNFalpha and IL1beta genes in microglial cells 24 hours after exposure in a dose-effect relationship **(****Figure 5A to** 5C). Levetiracetam did not modify IL6 gene expression after low-dose LPS challenge, but increased IL6 gene expression after a high-dose-LPS challenge in high-dose levetiracetam exposed cells (0.61 fold [0.25-0.97], p<0.001). Levetiracetam increased TNFalpha gene expression at a low LPS, low levetiracetam concentration (0.53; [0.82-0.26]; p<0.0001). The concentration of levetiracetam showed opposite effects at high LPS concentration, decreased TNFalpha gene expression at low dose (0.38 [0.10-0.66]; p<0.01) and increased its expression at high dose (0.58 [0.86-0.30]; p<0.0001), No effect of levetiracetam was observed on IL1 beta expressionin *vitro* in activated microglial cell or not.

### Microglial cell polarization

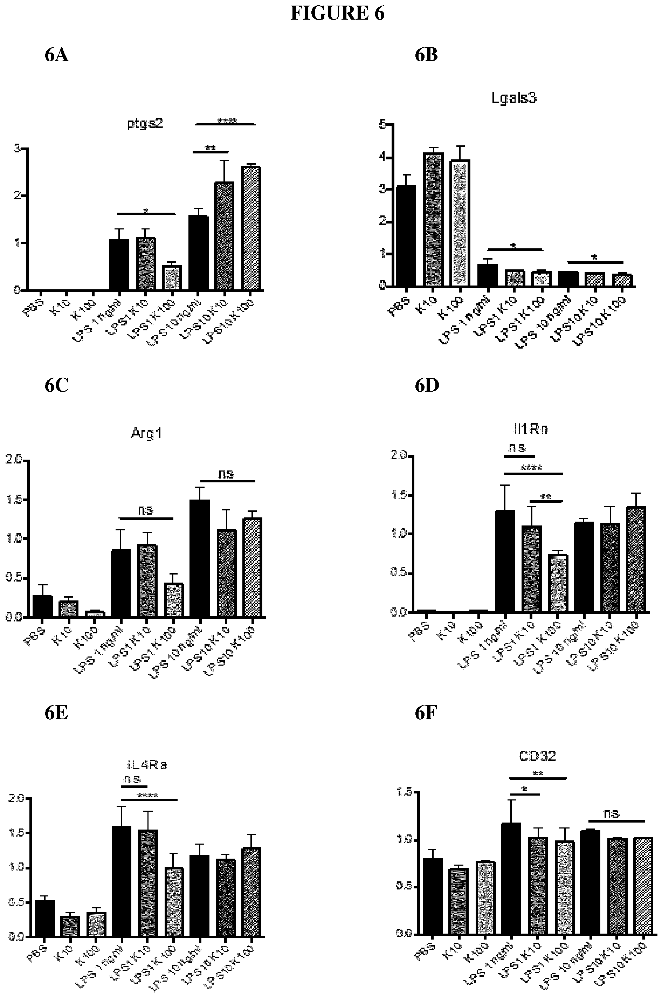

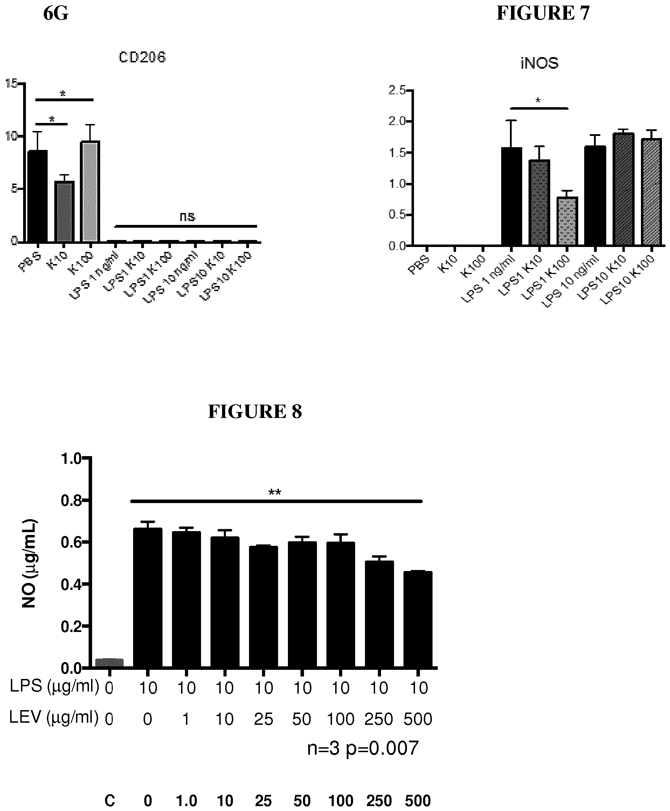

After LPS exposure, primary microglial cells show an increased expression of both pro and anti-inflammatory markers. Levetiracetam exposure modified the expression of both anti and proinflammatory markers (**Figure 6A to 6H**). Levetiracetam might modulate microglial polarization *in vivo* and could modulate microglial cell polarization *in vitro.*

### Nitric oxide production

The effect of levetiracetam on Inducible NO synthase gene (*inos*) transcription in activated primary murine microglial cells is shown in **Figure** 7. LPS challenge increased *inos* expression in a same extent whether a high-dose or a low-dose 24 hours LPS challenge was performed. Only high dose levetiracetam lowered *inos* expression in low dose LPS challenge but failed to modulate *inos* expression in high dose LPS challenge.

The effect of levetiracetam on Nitric Oxide production in murine primary monocytic cells exposed to LPS is shown in **Figure 8****.** LPS exposure increased NO production at a dose of 10 µg/mL after 24 exposure in cell culture supernatant. Levetiracetam reduced with a dose effect relationship the production of Nitric Oxide by activated murine monocytic cells. Levetiracetam reduce the production of NO through a reduction of expression of inducible Nitric Oxide Synthase.

### Discussion

The examples provide experimental evidence that levetiracetam acts to decrease mortality and symptoms related to posttraumatic stress disorder after sepsis. The results also indicate that levetiracetam can act on microglial cells and modulate regioally microglial activation. The results indicate that levetiracetam can modulate directly microglial cell activation.

Without wishing to be bound by theory, it is also possible that levetiracetam can reduce mortality through different mechanisms.

First levetiracetam may act direclty on amygdala neurons. Levetiracetam may also act indirectly on other groups of neurons, activator of the central amygdala (basolateral amygdala, periaqueduqual grey matter, vagal nerve nuclei).

Through its effects on neurotransmission, levetiracetam has been shown to lower calcium release from endoplasmic reticulum or to lower calcium through transmembranar channel. During sepsis intracellular calcium concentration is increased and might lead to apoptosis. On the opposite levetiracetam might protect against calcium induced induction of caspase 3 cleavage. Also its direct effect on mitochondrial cell might protect mitochondria to calcium overload and delay the opening of mitochondrial permeability transition pore.

The effect of levetiracetam might be also mediated by other cell types. First Sv2a mRNA expression has been observed in other cell types such as oligodendrocytes or microglial cells. SV2a has also been evidenced in mitochondria from brain cells. It has been shown that sepsis might act on mitochondrial metabolism. Levetiracetam has been shown to ameliorate the mitochondrial status in stress conditions (alzheimer's disease). It is thus possible that levetiracetam might act on sepsis induced mitochondrial dysfunction. It can also be supposed that microglial effect of levetiracetam might be mediate by its effect on mitochondria. This could explain a more reactive phenotype of microglial cell that can produce more energy to adopt a proinflammatory phenotype. Also it could be supposed that despite a higher neuroinflammatory and more excitotoxic environment, neurons are protected from an excitotoxic insult notably by a reduction in NO production by microglial cells

Sepsis is accompanied by different behavioural changes. The results presented herein provide evidence of an altered reactivity of amygdala in the fear conditioning. Sepsis seems to promote innate anxiety in the open field test but not in the elevated plus maze test. Also fear memory is more obviously increased during sepsis. It appears that amygdalar reaction in a very stressful context is different in mice that developed a sepsis. Fear memory is specially increased when the test depends solely on the amygdala and not the hippocapo-amygdalar complex. An important effect on the amygdala can be speculated. Indeed 6 hours after sepsis an important activation occurs in the central amygdala. In human dead from septic shock amygdala is a site of excitotoxicity and microglial apotosis.

Hippocampal dependent tests showed that sepsis might alter hippocampal functions. Long term reference memory implied different areas than the working memory. In the present study working memory seems preserved as mice were able to find the platform during the morris water maze but the long term memory consolidation might be slightly altered (probe trial, novel object location, novel object recognition). Areas in the working memory (dentate gyrus) are different than areas implied in the long term storage of memories (CA1,CA3 or the prefrontal cortex). An histological analysis of these areas might be of importance in the context. Also electrophysiological studies might show impairment in long-term potentiation in these areas. Long term memory requires sleep and synchronization between brain structures that induce brain rhythm changes (active, slight sleep, deep sleep, paradoxal sleep). The diminution of electrocorticographic rythms changes evidenced by a lower standard deviation might reflect the impairment in long term memory consolidation after sepsis and lower rhythm changes.

Interestingly, it was found that levetiracteam decreased the amygdala microglial activation concomitant to the neuronal activation. A direct effect of levetiracetam on primary microglial cell culture was observed. However, such a poperty has never been reported. It might be mediated by an action on SV2a or consist in an effect off the target, due to a decrease of antiSV2a specific with the high dose of levetiracetam used in the present study. It is more plausible that levetiracetam microglial effect is indirect, refleting the decrease in neuronal activation.

Microglial cell sorting in whole brain of septic mice receiving levetiracetam showed a surprising result: microglial cells were polarized towards a pro-inflammatory (M1) phenotype.

Microglial cell polarization was modified with sepsis and levetiracetam might modulate microglial cell activation in a region specific way.

It was also found that levetiracetam reduced nitric oxid production.

## Claims

1. A synaptic vesicle 2a binding chemical entity for use in the prevention and/or treatment of sepsis induced acute brain dysfunction (SiBD), wherein the synaptic vesicle 2a binding chemical entity is levetiracetam, brivaracetam or selectracetam.

2. The synaptic vesicle 2a binding chemical entity for use according to claim 1, wherein said levetiracetam, brivaracetam or selectracetam increases the cerebral level of IL1β or IL4, or hippocampal expression of lba-1 in the subject, and/or reduces cleavage of Caspase 3 in the brain of the subject.

3. The synaptic vesicle 2a binding chemical entity for use according to claim 1 or claim 2, wherein the synaptic vesicle 2a binding chemical entity is levetiracetam.

4. The synaptic vesicle 2a binding chemical entity for use according to any one of claims 1 to 3, wherein the synaptic vesicle 2a binding chemical entity is administered every twelve hours.

5. The synaptic vesicle 2a binding chemical entity for use according to any one of claims 1 to 4, wherein the synaptic vesicle 2a binding chemical entity is administered for at least two or four days.

6. The synaptic vesicle 2a binding chemical entity for use according to claim 5, wherein the synaptic vesicle 2a binding chemical entity is administered for at least one, two or four weeks.

7. The synaptic vesicle 2a binding chemical entity for use according to any one of claims 1 to 6, wherein administration of the synaptic vesicle 2a binding chemical entity to the subject reduces development of at least one cognitive indicator of SiBD or reduces development of post traumatic stress disorder induced by SiBD in the subject.

8. The synaptic vesicle 2a binding chemical entity for use according to any one of claims 1 to 6, wherein administration of the synaptic vesicle 2a binding chemical entity to the subject reduces the risk of sepsis induced death or reduces the risk of sepsis induced dementia in the subject.

9. A synaptic vesicle 2a binding chemical entity for use for reducing sepsis mortality, wherein the synaptic vesicle 2a binding chemical entity is levetiracetam, brivaracetam or selectracetam, and wherein the synaptic vesicle 2a binding chemical entity is administered for at least one week.

10. The synaptic vesicle 2a binding chemical entity for use according to claim 9, wherein said levetiracetam, brivaracetam or selectracetam increases the cerebral level of IL1β or IL4, or hippocampal expression of lba-1 in the subject, and/or reduces cleavage of Caspase 3 in the brain of the subject.

11. The synaptic vesicle 2a binding chemical entity for use according to claim 9 or 10, wherein the synaptic vesicle 2a binding chemical entity is levetiracetam.

12. The synaptic vesicle 2a binding chemical entity for use according to any one of claims 9 to 11, wherein the synaptic vesicle 2a binding chemical entity is administered every twelve hours.

13. The synaptic vesicle 2a binding chemical entity for use according to any one of claims 9 to 12, wherein the synaptic vesicle 2a binding chemical entity is administered for at least two or four weeks.

## Patentansprüche

1. Ein synaptisches Vesikel 2a bindende chemische Einheit zum Verwenden beim Vorbeugen und/oder Behandeln von Sepsis-induzierter akuter Hirnfunktionsstörung (SiBD), wobei die ein synaptisches Vesikel 2a bindende chemische Einheit Levetiracetam, Brivaracetam oder Selectracetam ist.

2. Ein synaptisches Vesikel 2a bindende chemische Einheit zum Verwenden gemäß Anspruch 1, wobei das Levetiracetam, Brivaracetam oder Selectracetam den zerebralen Spiegel von IL1β oder IL4 oder die hippocampale Expression von lba-1 in dem Probanden erhöht und/oder die Spaltung von Caspase 3 im Gehirn des Probanden reduziert.

3. Ein synaptisches Vesikel 2a bindende chemische Einheit zum Verwenden gemäß Anspruch 1 oder Anspruch 2, wobei die ein synaptische Vesikel 2a bindende chemische Einheit Levetiracetam ist.

4. Ein synaptisches Vesikel 2a bindende chemische Einheit zum Verwenden nach einem der Ansprüche 1 bis 3, wobei die ein synaptisches Vesikel 2a bindende chemische Einheit alle zwölf Stunden verabreicht wird.

5. Ein synaptisches Vesikel 2a bindende chemische Einheit zum Verwenden nach einem der Ansprüche 1 bis 4, wobei die ein synaptisches Vesikel 2a bindende chemische Einheit über mindestens zwei oder vier Tage verabreicht wird.

6. Ein synaptisches Vesikel 2a bindende chemische Einheit zum Verwenden gemäß Anspruch 5, wobei die eine synaptische Vesikel 2a bindende chemische Einheit über mindestens eine, zwei oder vier Wochen verabreicht wird.

7. Ein synaptisches Vesikel 2a bindende chemische Einheit zum Verwenden nach einem der Ansprüche 1 bis 6, wobei das Verabreichen der ein synaptisches Vesikel 2a bindenden chemischen Einheit an den Probanden die Entwicklung von mindestens einem kognitiven Indikator von SiBD reduziert oder die Entwicklung einer durch SiBD induzierten posttraumatischen Belastungsstörung in dem Probanden reduziert.

8. Ein synaptisches Vesikel 2a bindende chemische Einheit zum Verwenden nach einem der Ansprüche 1 bis 6, wobei das Verabreichen der ein synaptisches Vesikel 2a bindenden chemischen Einheit an den Probanden das Risiko eines Sepsis-induzierten Todes oder das Risiko einer Sepsis-induzierten Demenz in dem Probanden verringert.

9. Ein synaptisches Vesikel 2a bindende chemische Einheit zum Verwenden zur Verringerung der Sepsis-Sterblichkeit, wobei die ein synaptisches Vesikel 2a bindende chemische Einheit Levetiracetam, Brivaracetam oder Selectracetam ist und wobei die ein synaptisches Vesikel 2a bindende chemische Einheit über mindestens eine Woche verabreicht wird.

10. Ein synaptisches Vesikel 2a bindende chemische Einheit zum Verwenden gemäß Anspruch 9, wobei das Levetiracetam, Brivaracetam oder Selectracetam den zerebralen Spiegel von IL1β oder IL4 oder die hippocampale Expression von lba-1 in dem Probanden erhöht und/oder die Spaltung von Caspase 3 im Gehirn des Probanden reduziert.

11. Ein synaptisches Vesikel 2a bindende chemische Einheit zum Verwenden gemäß Anspruch 9 oder 10, wobei die ein synaptisches Vesikel 2a bindende chemische Einheit Levetiracetam ist.

12. Ein synaptische Vesikel 2a bindende chemische Einheit zum Verwenden gemäß einem der Ansprüche 9 bis 11, wobei die ein synaptisches Vesikel 2a bindende chemische Einheit alle zwölf Stunden verabreicht wird.

13. Ein synaptisches Vesikel 2a bindende chemische Einheit zum Verwenden gemäß einem der Ansprüche 9 bis 12, wobei die ein synaptisches Vesikel 2a bindende chemische Einheit über mindestens zwei oder vier Wochen verabreicht wird.

## Revendications

1. Entité chimique liant la vésicule synaptique 2a destinée à être utilisée dans la prévention et/ou le traitement du dysfonctionnement cérébral aigu induit par la septicémie (SiBD), dans laquelle l'entité chimique liant la vésicule synaptique 2a est le lévétiracétam, le brivaracétam ou le sélectracétam.

2. Entité chimique liant la vésicule synaptique 2a destinée à être utilisée selon la revendication 1, dans laquelle ledit lévétiracétam, brivaracétam ou sélectracétam augmente le niveau cérébral d'IL1β ou d'IL4, ou l'expression hippocampique de lba-1 chez le sujet, et/ou réduit le clivage de Caspase 3 dans le cerveau du sujet.

3. Entité chimique liant la vésicule synaptique 2a destinée à être utilisée selon la revendication 1 ou la revendication 2, dans laquelle l'entité chimique liant la vésicule synaptique 2a est le lévétiracétam.

4. Entité chimique liant la vésicule synaptique 2a destinée à être utilisée selon l'une quelconque des revendications 1 à 3, dans laquelle l'entité chimique liant la vésicule synaptique 2a est administrée toutes les douze heures.

5. Entité chimique liant la vésicule synaptique 2a destinée à être utilisée selon l'une quelconque des revendications 1 à 4, dans laquelle l'entité chimique liant la vésicule synaptique 2a est administrée pendant au moins deux ou quatre jours.

6. Entité chimique liant la vésicule synaptique 2a destinée à être utilisée selon la revendication 5, dans laquelle l'entité chimique liant la vésicule synaptique 2a est administrée pendant au moins une, deux ou quatre semaines.

7. Entité chimique liant la vésicule synaptique 2a destinée à être utilisée selon l'une quelconque des revendications 1 à 6, dans laquelle l'administration de l'entité chimique liant la vésicule synaptique 2a au sujet réduit le développement d'au moins un indicateur cognitif de SiBD ou réduit le développement d'un trouble de stress post-traumatique induit par SiBD chez le sujet.

8. Entité chimique liant la vésicule synaptique 2a destinée à être utilisée selon l'une quelconque des revendications 1 à 6, dans laquelle l'administration de l'entité chimique liant la vésicule synaptique 2a au sujet réduit le risque de décès induit par la septicémie ou réduit le risque de démence induit par la septicémie chez le sujet.

9. Entité chimique liant la vésicule synaptique 2a destinée à être utilisée pour réduire la mortalité due à la septicémie, dans laquelle l'entité chimique liant la vésicule synaptique 2a est le lévétiracétam, le brivaracétam ou le sélectracétam, et dans laquelle l'entité chimique liant la vésicule synaptique 2a est administrée pendant au moins une semaine.

10. Entité chimique liant la vésicule synaptique 2a destinée à être utilisée selon la revendication 9, dans laquelle ledit lévétiracétam, brivaracétam ou sélectracétam augmente le niveau cérébral d'IL1β ou d'IL4, ou l'expression hippocampique de lba-1 chez le sujet, et/ou réduit le clivage de Caspase 3 dans le cerveau du sujet.

11. Entité chimique liant la vésicule synaptique 2a destinée à être utilisée selon la revendication 9 ou 10, dans laquelle l'entité chimique liant la vésicule synaptique 2a est le lévétiracétam.

12. Entité chimique liant la vésicule synaptique 2a destinée à être utilisée selon l'une quelconque des revendications 9 à 11, dans laquelle l'entité chimique liant la vésicule synaptique 2a est administrée toutes les douze heures.

13. Entité chimique liant la vésicule synaptique 2a destinée à être utilisée selon l'une quelconque des revendications 9 à 12, dans laquelle l'entité chimique liant la vésicule synaptique 2a est administrée pendant au moins deux ou quatre semaines.
